(19) Europäisches Patentamt / European Patent Office / Office européen des brevets

(11) **EP 4 252 905 A1**

# EUROPEAN PATENT APPLICATION
published in accordance with Art. 153(4) EPC

(43) Date of publication:
04.10.2023 Bulletin 2023/40

(21) Application number: 21897408.7

(22) Date of filing: 03.08.2021

(51) International Patent Classification (IPC):
*B01J 23/89* (2006.01)    *B01J 37/02* (2006.01)
*B01J 37/16* (2006.01)    *C07C 67/055* (2006.01)
*C07C 69/15* (2006.01)    *C07B 61/00* (2006.01)

(52) Cooperative Patent Classification (CPC):
**B01J 23/89; B01J 37/02; B01J 37/16;**
**C07C 67/055; C07C 69/15;** C07B 61/00

(86) International application number:
**PCT/JP2021/028834**

(87) International publication number:
**WO 2022/113429 (02.06.2022 Gazette 2022/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 27.11.2020 JP 2020196989

(71) Applicant: Resonac Corporation
Tokyo 105-8518 (JP)

(72) Inventors:
• **KITAGAWA, Kazuhiro**
Oita-shi, Oita 870-0189 (JP)
• **HOSOGI, Yasuhiro**
Oita-shi, Oita 870-0189 (JP)
• **IWAMA, Yasuhiro**
Oita-shi, Oita 870-0189 (JP)
• **UMEHARA, Kazuki**
Oita-shi, Oita 870-0189 (JP)

(74) Representative: **Strehl Schübel-Hopf & Partner
Maximilianstrasse 54
80538 München (DE)**

(54) **METHOD FOR MANUFACTURING CATALYST FOR MANUFACTURE OF VINYL ACETATE AND METHOD FOR MANUFACTURING VINYL ACETATE**

(57) Provided is a method for manufacturing a catalyst with which it is possible to manufacture vinyl acetate at a remarkably high selection rate while ensuring a high catalyst activity. A method for manufacturing a catalyst for manufacture of vinyl acetate containing a carrier, copper, palladium, gold, and an acetate, the method comprising in the following order: step 1) a step for impregnating the carrier with an alkaline solution; step 2) a step for contact-impregnating the carrier with a solution containing a compound containing copper, a compound containing palladium, and a compound containing gold; step 3) a step for performing reduction treatment; and step 4) a step for causing the carrier to carry the acetate.

EP 4 252 905 A1

**Description**

FIELD

The present invention relates to a method for producing a vinyl acetate production catalyst which is used in the production of vinyl acetate using acetic acid, ethylene, and oxygen as raw materials, as well as a method for producing vinyl acetate using such a catalyst.

BACKGROUND

Vinyl acetate is used as a raw material for vinyl acetate resins, a raw material for polyvinyl alcohol, and a monomer for copolymerization with ethylene, styrene, acrylates, and methacrylates in a wide range of fields such as paints, adhesives, and textile treatment agents and is an important industrial material.

A catalyst of palladium, gold, and potassium acetate supported on silica is widely used for the production of vinyl acetate using acetic acid, ethylene, and oxygen as raw materials. The active site in this reaction is considered to be the palladium, and gold is thought to play a role in, in addition to suppressing palladium aggregation, reducing the production of carbon dioxide, which is a by-product, thereby improving vinyl acetate selectivity. In order for this effect of gold to be, gold atoms must be present in close proximity to the palladium. In Patent Literature 1, by devising a process for impregnating the carrier, palladium and gold are supported in a state in which the supported locations thereof are near each other.

In the production of vinyl acetate, increasing vinyl acetate selectivity is an important technical object, and from the viewpoint of environmental impact, it is desirable to suppress the generation of carbon dioxide gas.

In Patent Literature 2, vinyl acetate selectivity is improved by supporting copper in addition to palladium and gold.

[CITATION LIST]

[PATENT LITERATURE]

[PTL 1] JP 2008-080326 A
[PTL 2] JP 2002-516749 A

SUMMARY

[TECHNICAL PROBLEM]

In Patent Literature 2, a palladium-containing compound, a gold-containing compound, and a copper-containing compound are supported on a carrier in separate steps, and the influence of the copper-containing compound in the supporting step on the ultimate states of the metallic palladium and the metallic gold is much smaller as compared to the case in which they are supported in the same step. It has not been known that a catalyst is produced by supporting a palladium-containing compound, a gold-containing compound, and a copper-containing compound on a carrier in the same step, and the thus-obtained catalyst is used, whereby vinyl acetate selectivity is improved.

An object of the present invention is to provide a method for producing a catalyst with which vinyl acetate can be produced with an exceptionally high selectivity while ensuring high catalyst activity.

[SOLUTION TO PROBLEM]

As a result of extensive research in order to solve the above problems, the present inventors have discovered a method for producing a catalyst wherein in addition to a palladium-containing compound and a gold-containing compound, a copper-containing compound is supported in the same step while reacting with an alkaline component, and have succeeded in producing vinyl acetate with exceptionally high selectivity. Gold-containing compounds represented by chloroauric acid are generally hydrolyzed slowly, and are supported on carriers at a slow rate. However, if a copper-containing compound is co-present during the hydrolysis of a gold-containing compound, the copper-containing compound is rapidly hydrolyzed, whereby the copper-containing compound supported by the carrier adsorbs the gold-containing compound in the solution. As a result, the gold-containing compound stays in the solution for a short time, whereby the gold can be atomized compared to the case where the copper-containing compound is not added in the same step. Atomized gold contributes significantly to improvement of vinyl acetate selectivity. The catalyst produced by the method of supporting the copper-containing compound in a step different from the steps of supporting the palladium-containing compound and the gold-containing compound described in Patent Literature 2 did not exhibit an exceptionally high selectivity, unlike in the present invention.

Specifically, the present invention encompasses [1] to [6] below.

[1] A method for producing a vinyl acetate production catalyst comprising a carrier, copper, palladium, gold, and an acetate, the method comprising the following steps in this order:

step 1: impregnating a carrier with an alkaline solution,
step 2: contacting and impregnating the carrier with a solution containing a copper-containing compound, a palladium-containing compound, and a gold-containing compound,
step 3: performing a reduction treatment, and
step 4: supporting the acetate on the carrier.

[2] The method according to [1], wherein a mass of supported metallic copper per kg of catalyst is 0.1 g or more and 1.6 g or less.
[3] The method according to [1] or [2], wherein a mass of supported metallic palladium per kg of catalyst is 8.0 g or more and 16.0 g or less.
[4] The method according to any of [1] to [3], wherein a mass of supported metallic gold per kg of catalyst is 4.0 g or more and 12.0 g or less.
[5] The method according to any of [1] to [4], wherein a mass of supported acetate per kg of catalyst is 40 g or more and 100 g or less.
[6] A method for producing vinyl acetate using ethylene, oxygen, and acetic acid as raw materials, wherein a vinyl acetate production catalyst obtained by the method according to any of [1] to [5] is used.

[ADVANTAGEOUS EFFECTS OF INVENTION]

According to the method of the present invention, a vinyl acetate production catalyst in which copper, palladium, and gold are supported on a carrier can easily be produced, and vinyl acetate selectivity can be remarkably improved while ensuring high catalyst activity, as compared to conventional methods.

BRIEF DESCRIPTION OF DRAWINGS

FIG. 1 is a graph showing the relationship between the supported amount of copper and vinyl acetate selectivity in the Examples and Comparative Examples.
FIG. 2 is a graph showing the relationship between the supported amount of gold and vinyl acetate selectivity in the Examples and Comparative Examples.

DESCRIPTION OF EMBODIMENTS

The embodiments of the present invention will be described below, but the present invention is not limited to only these embodiments, and various changes can be made within the scope of implementation of the present invention.

[Method for Producing Vinyl Acetate Production Catalyst]

The method for producing a vinyl acetate production catalyst according to an embodiment comprising the following steps in this order:

Step 1: impregnating a carrier with an alkaline solution,
Step 2: contacting and impregnating the carrier with a solution (hereinafter referred to as "solution A") containing a copper-containing compound, a palladium-containing compound, and a gold-containing compound,
Step 3: performing a reduction treatment, and
Step 4: supporting the acetate on the carrier.

In an embodiment, step 2 is performed after step 1, and the carrier is contacted and impregnated with solution A containing the copper-containing compound, the palladium-containing compound, and the gold-containing compound to form a catalyst precursor in which these compounds are supported on the carrier.

Though steps 1 to 4 are preferably performed in the order described above, other steps may be included in order to improve catalyst performance. Solution A may contain other components. Since the reduction treatment of step 3 is for converting the copper-containing compound, the palladium-containing compound, and the gold-containing compound to metallic copper, metallic palladium, and metallic gold, respectively, it must be carried out after step 2. Each step will be described in detail below.

<Step 1. Impregnating Carrier with Alkaline Solution>

In this step, the carrier is impregnated with the alkaline solution. This step can be performed at room temperature. After the impregnation operation is completed, the carrier may be dried or may undergo the next step without undergoing drying or other operations.

The carrier is not particularly limited, and a porous substance which is generally used as a carrier for catalysts can be used. The carrier is preferably silica, alumina, silica-alumina, diatomaceous earth, montmorillonite, or titania, and more preferably silica. When a carrier containing silica as a main component is used, the silica content of the carrier is generally at least 50% by mass, and preferably at least 90% by mass, based on the mass of the carrier.

The specific surface area of the carrier as measured by the BET method is preferably at least 0.01 $m^2$/g, more preferably in the range of 10 to 1000 $m^2$/g, and particularly preferably in the range of 100 to 500 $m^2$/g. The bulk density of the carrier is preferably in the range of 50 to 1000 g/L, and particularly preferably in the range of 300 to 500 g/L. The water absorption of the carrier is preferably in the range of 0.05 to 3 g-water/g-carrier, and particularly preferably in the range of 0.1 to 2 g-water/g-carrier. Regarding the pore structure of the carrier, the average pore diameter is preferably in the range of 1 to 1000 nm, and particularly preferably in the range of 2 to 800 nm. When the average pore diameter is 1 nm or more, diffusion of gas can be facilitated. Conversely, when the average pore diameter is 1000 nm or less, the specific surface area of the carrier necessary for obtaining catalyst activity can be ensured.

A mercury intrusion method and a gas adsorption method (BJH method) are widely used to measure the pore size distribution of the carrier. With reference to the pore classification of the IUPAC (International Union of Pure and Applied Chemistry), the mercury intrusion method can measure macropores of 50 nm or more and some mesopores of 2 nm to less than 50 nm, and the gas adsorption method can measure mesopores and micropores of 2 nm or less. The appropriate measuring method can be selected in accordance with the size of the pore diameter.

In the present disclosure, the water absorption of the carrier refers to the numerical value measured by the following measurement method.

    1. Approximately 5 g of the carrier is weighed (W1 g) with a balance and placed in a 100 mL beaker.
    2. Approximately 15 mL of pure water (ion-exchanged water) is added to the beaker so as to completely cover the carrier.
    3. The beaker is allowed to stand for 30 minutes.
    4. The contents of the beaker are placed on a wire mesh to drain the pure water.
    5. Water adhering to the surface of the carrier is removed by lightly pressing with a paper towel until the surface becomes dull.
    6. The total mass of carrier and pure water is measured (W2 g).
    7. The water absorption of the carrier is calculated by the following formula:

$$\text{Water absorption (g-water/g-carrier)} = (W2 - W1) / W1$$

Thus, the water absorption amount of the carrier (g) is calculated by multiplying the water absorption of the carrier (g-water/g-carrier) × the mass of the carrier used (g).

The form of the carrier is not particular limited. Specific examples include powder-like, spherical, and pelletized, but the form is not limited thereto. The optimal form can be selected in accordance with the reaction type and reaction vessel used.

The size of the carrier particles is not particularly limited. When the carrier is spherical, the particle diameter thereof is preferably in the range of 1 to 10 mm, and more preferably in the range of 3 to 8 mm. In the case of a gas phase reaction in a tubular reactor filled with a catalyst, when the particle diameter is 1 mm or more, excessive increases in pressure loss during gas flow can be prevented, whereby the gas can effectively be circulated. Conversely, when the particle diameter is 10 mm or less, the raw material gas can be easily diffused into the interior of the catalyst, whereby the catalyst reaction can proceed effectively. Furthermore, since the number of catalyst particles packed into the tubular reactor does not decrease excessively, sufficient total surface area of the catalyst particles can be ensured to make the amounts of metal components (copper, palladium, and gold) dispersed on the surface of the carrier appropriate for the reaction.

As the alkaline solution, any alkaline compound can be used. Examples of the alkaline compound include alkali metal or alkaline earth metal hydroxides, alkali metal or alkaline earth metal bicarbonates, alkali metal or alkaline earth metal carbonates, and alkali metal or alkaline earth metal silicates. Lithium, sodium, or potassium can be used as the alkali metal. Barium or strontium can be used as the alkaline earth metal. Sodium metasilicate, potassium metasilicate, sodium hydroxide, potassium hydroxide, barium hydroxide, or strontium hydroxide is preferably used as the alkaline compound.

The solvent of the alkaline solution is not particularly limited, examples thereof include water, methanol, and ethanol, and water is preferable.

The alkaline compound is used in excess relative to the total of the copper, palladium, and gold, which are described later. For example, the product of the molar amount of the alkaline compound and the valence of the alkaline compound is preferably greater than 1.1-fold and 3.0-fold or less, and more preferably greater than 1.5-fold and 2.0-fold or less the sum of the product of the molar amount of the palladium-containing compound and the valence of palladium, the product of the molar amount of the gold-containing compound and the valence of gold, and the product of the molar amount of the copper-containing compound and the valence of copper.

The method for impregnating the carrier with the alkaline solution is not particularly limited. Examples thereof include (I) a method of immersing the carrier in a large amount of alkaline solution for some time and thereafter removing the carrier impregnated with the alkaline solution corresponding to the water absorption amount; and (II) a method of dissolving an alkaline compound in a solvent and diluting it to a level equivalent to the water absorption amount of the carrier, and thereafter impregnating the carrier with it. Method (II) is preferable from the viewpoint of waste liquid treatment.

The carrier is impregnated with the alkaline solution having an amount equivalent to, preferably, 0.9-fold or more and 1.0-fold or less the mass of the water absorption amount of the carrier, and more preferably, 0.95-fold or more and 1.0-fold or less the mass of the water absorption amount of the carrier. When the amount of alkaline solution is 0.9-fold or more the mass of the water absorption amount of the carrier, impregnation disparity of the alkaline solution is less likely to occur. When the amount of alkaline solution is 1.0-fold or less the mass of the water absorption amount of the carrier, the entire amount of alkaline solution can be reliably absorbed by the carrier. In the present disclosure, the water absorption amount of the carrier is a value measured with pure water, which is strictly different from the value for alkaline solution, but is used as-is for the sake of convenience.

<Step 2. Step of Contacting and Impregnating Carrier with Solution A>

In this step, the carrier, which has been impregnated with alkaline solution, is brought into contact and impregnated with solution A. Solution A is a solution containing the copper-containing compound, the palladium-containing compound, and the gold-containing compound. Other components may be dissolved in solution A as needed.

The raw material compound of each catalyst component contained in solution A is adjusted so as to achieve the desired catalyst composition. The concentration of the raw material compound of each of the catalyst components (copper, palladium, and gold) in solution A can be calculated from the amount of the raw material compound to be supported on the carrier and the amount of the solution. In actual operations, the amount (g) of raw material compound to be supported on the carrier is weighed and dissolved in a solvent so as to achieve the desired amount of solution.

As the copper-containing compound in solution A, a copper precursor which can be converted into metallic copper can be used. Copper precursors which can be converted into metallic copper include, for example, copper chloride, copper acetate, and copper nitrate, and copper chloride is preferably used.

As the palladium-containing compound in solution A, a palladium precursor which can be converted into metallic palladium can be used. Examples of palladium precursors which can be converted into metallic palladium include palladium chloride, palladium nitrate, palladium sulfate, sodium chloropalladate, potassium chloropalladate, barium chloropalladate, and palladium acetate, and sodium chloropalladate is preferably used.

As the gold-containing compound in solution A, a gold precursor which can be converted into metallic gold can be used. Examples of gold precursors include chloroauric acid, sodium chloroaurate, and potassium chloroaurate, and chloroauric acid is preferably used.

Examples of the solvent of solution A include water, alcohols, and organic acids. Water is preferable, because it does not damage the carrier and does not react with the compounds contained in solution A.

The amount of solution A is preferably 1.0 to 10.0-fold, more preferably 2.0 to 8.0-fold, and particularly preferably 2.0 to 5.0-fold the mass of the water absorption amount of the carrier.

By bringing the carrier, which has been impregnated with the alkaline solution, into contact with solution A, the raw material metal compounds can be converted into water-insoluble substances to form a shell-type catalyst precursor in which the metal components such as palladium, gold, and copper are unevenly distributed and supported on the surface portion of the carrier.

The contact time is not particularly limited, and is preferably 0.5 to 100 hours, and more preferably 3 to 50 hours. By setting the contact time to 0.5 hours or more, a desired amount of the catalyst component can be supported, and sufficient catalyst performance can be obtained. By setting the contact time to 100 hours or less, degradation of the carrier can be suppressed.

The contact temperature is not particularly limited, and is preferably 10 to 80°C, and more preferably 20 to 60°C. By setting the contact temperature to 10°C or higher, the conversion reaction can sufficiently advance. By setting the contact temperature to 80°C or lower, aggregation of the copper, palladium, and gold can be suppressed.

<Step 3. Step of Performing Reduction Treatment>

It is desirable that the carrier supporting the copper-containing compound (copper chloride, etc.), the palladium-containing compound (palladium salt, etc.), and the gold-containing compound (chloroauric acid, etc.) be subjected to reduction treatment to convert the compounds to metallic palladium, metallic gold, and metallic copper. In the present disclosure, the phrases "metallic copper", "metallic palladium", and "metallic gold" refer to metal species having a valence of zero. Reduction treatment can be either by liquid phase reduction or gas phase reduction. Not the entirety of the palladium, gold, and copper may be reduced to the metallic state, i.e., zero valence, after reduction treatment.

Liquid phase reduction can be carried out either in a non-aqueous system using an alcohol or hydrocarbon, or in an aqueous system. Examples of reducing agents which can be used include carboxylic acids and salts thereof, aldehydes, hydrogen peroxide, sugars, polyhydric phenols, boron compounds, amines, and hydrazine. Examples of carboxylic acids and salts thereof include oxalic acid, potassium oxalate, formic acid, potassium formate, potassium citrate, and ammonium citrate. Examples of aldehydes include formaldehyde and acetaldehyde. Examples of sugars include glucose. Examples of polyhydric phenols include hydroquinone. Examples of boron compounds include diborane and sodium borohydride. As the reducing agent, hydrazine, formaldehyde, acetaldehyde, hydroquinone, sodium borohydride, and potassium citrate are preferred, and hydrazine is particularly preferred.

When the liquid phase reduction is performed, the temperature is not particularly limited, and the liquid phase temperature is preferably in the range of 0 to 200°C, and more preferably in the range of 10 to 100°C. When the liquid phase temperature is 0°C or higher, a sufficient reduction rate can be obtained. Conversely, when the liquid phase temperature is 200°C or lower, aggregation of the copper, palladium, and gold can be suppressed. The reduction time is not particularly limited, and the reduction time is preferably in the range of 0.5 to 24 hours, and more preferably in the range of 1 to 10 hours. When the reduction time is 0.5 hours or longer, the reduction can sufficiently advance. Conversely, when the reduction time is 24 hours or less, aggregation of the copper, palladium, and gold can be suppressed.

The reducing agent used for gas phase reduction can be selected from hydrogen gas, carbon monoxide, alcohols, aldehydes, and olefins such as ethylene, propene, and isobutene. The reducing agent is preferably hydrogen gas. In gas phase reduction, an inert gas may be added as a diluent. Examples of inert gases include helium, argon, and nitrogen gases.

When gas phase reduction is performed, the temperature is not particularly limited, and the impregnated carrier is preferably heated to a range of 30 to 350°C, and more preferably to a range of 100 to 300°C. When the heating temperature is 30°C or higher, a sufficient reduction rate can be obtained. Conversely, when the heating temperature is 300°C or lower, aggregation of the copper, palladium, and gold can be suppressed. The reduction time is not particularly limited, and the reduction time is preferably in the range of 0.5 to 24 hours, and more preferably in the range of 1 to 10 hours. When the reduction time is 0.5 hours or longer, the reduction can sufficiently advance. Conversely, when the reduction time is 24 hours or less, aggregation of the copper, palladium, and gold can be suppressed.

The pressure of the gas phase reduction is not particularly limited, and is preferably in the range of 0.0 to 3.0 MPaG (gauge pressure) from the viewpoint of equipment, and more preferably in the range of 0.1 to 1.0 MPaG (gauge pressure).

The supply rate of the reducing agent when gas phase reduction is performed is preferably in the range of a space velocity (hereinafter referred to as SV) of 10 to 15,000 $hr^{-1}$, and particularly preferably 100 to 8,000 $hr^{-1}$ under standard conditions.

The carrier which has been subjected to reduction treatment is washed with pure water, if necessary. Washing may be performed continuously or in batches. The washing temperature is preferably in the range of 5 to 200°C, and more preferably in the range of 15 to 80°C. The washing time is not particularly limited, and conditions sufficient for the purpose of removing unfavorable residual impurities may be selected. Unwanted impurities include, for example, chlorine-containing compounds and chloride ions. After washing, the carrier may be dried by heating if necessary.

<Step 4. Step of Supporting Acetate on Carrier >

The acetate can be supported by impregnating the carrier with a solution containing the required amount of acetate and drying. The amount of the acetate solution to be used is preferably 0.9 to 1-fold the mass of the water absorption amount of the carrier. Acetate supporting is typically performed after reduction treatment, but can also be performed before reduction treatment.

The acetate is preferably at least one compound selected from alkali metal acetates and alkaline earth metal acetates, and more preferably alkali metal acetates. Examples of alkali metal acetates include lithium, sodium, and potassium acetates. Sodium acetate and potassium acetate are preferable as the acetate, and potassium acetate is particularly preferable.

[Vinyl Acetate Production Catalyst]

The mass of supported metallic copper per kg of catalyst (the sum of the carrier mass, catalyst metal mass, acetate mass, and the mass of the other components) is preferably 0.1 g or more and 1.6 g or less, more preferably 0.3 g or more and 1.4 g or less, and particularly preferably 0.6 g or more and 1.2 g or less.

The mass of supported metallic palladium per kg of catalyst is preferably 8.0 g or more and 16.0 g or less, and more preferably 10.0 g or more and 14.0 g or less.

The mass of supported metallic gold per kg of catalyst is preferably 4.0 g or more and 12.0 g or less, and more preferably 5.0 g or more and 10.0 g or less.

The mass of supported acetate per kg of catalyst is preferably 40 g or more and 100 g or less, and more preferably 50 g or more and 70 g or less.

The vinyl acetate production catalyst obtained by the method of the present disclosure has an eggshell structure in which a large portion of the copper, palladium, and gold are supported on the surface portion of the carrier. The thickness of the shell portion varies, depending on the type of carrier, alkaline solution, and raw metal compound aqueous solution used. When spherical silica having a diameter of 5 mm is used as the carrier, the shell portion preferably has a thickness of 0.05 to 0.5 mm, and more preferably 0.1 to 0.3 mm. When the thickness of the shell portion is 0.05 mm or more, catalyst activity can be maintained even if the surface portion of the carrier is peeled off during the reaction. When the thickness of the shell portion is 0.5 mm or less, the concentration of the catalyst on the surface portion of the carrier can be sufficiently increased, whereby the shell-type support can bring the economic benefits. The acetate not need be supported in shell form, and may be present uniformly throughout the catalyst.

[Specific Example of Catalyst Production]

A specific examples of catalyst production is shown below.

1. The carrier is impregnated with the alkaline solution in an amount corresponding to the water absorption amount of the carrier.
2. The carrier is immersed in solution A obtained by diluting the raw material metal compounds of copper, palladium, and gold with pure water to twice the mass of the water absorption amount of the carrier to impregnate the carrier and form a catalyst precursor.
3. Reduction treatment is performed by adding a reducing agent to the dispersion containing the catalyst precursor obtained in step 2.
4. The catalyst precursor after reduction is washed with pure water.
5. The washed catalyst precursor is dried.
6. A predetermined amount of an acetate is supported thereon.
7. The catalyst-supported carrier is washed.

[Production of Vinyl Acetate]

A method for producing vinyl acetate using the vinyl acetate production catalyst produced by the method of the present disclosure will be described below. The reaction for producing vinyl acetate uses acetic acid, ethylene, and oxygen as reactants, and is preferably carried out in a gas phase. The gas phase reaction may be in any conventionally known form, and is preferably a fixed bed flow reaction.

The reaction formula is as follows.

$$CH_2=CH_2 + CH_3COOH + 1/2O_2 \rightarrow CH_2=CHOCOCH_3 + H_2O$$

The ratio of acetic acid, ethylene, and oxygen in the raw material gas is preferably acetic acid : ethylene : oxygen = 1:0.08 to 16:0.01 to 4 as a molar ratio, and more preferably, acetic acid : ethylene : oxygen = 1:0.2 to 9:0.07 to 2.

The raw material gas contains ethylene, acetic acid (gas), and oxygen gas, and may further contain nitrogen gas, carbon dioxide, or a noble gas as a diluent as necessary. When ethylene, acetic acid, and oxygen are defined as reaction raw materials, the ratio of the reaction raw materials and the diluent is preferably a molar ratio of reaction raw materials : diluent = 1:0.05 to 9, and more preferably reaction raw materials : diluent = 1:0.1 to 3.

When the reaction is performed in a fixed bed flow reaction, the raw material gas is preferably supplied to the reactor at a space velocity (SV) of 10 to 15,000 $hr^{-1}$, and more preferably 300 to 8,000 $hr^{-1}$ under standard conditions. By setting the space velocity to 10 $hr^{-1}$ or more, the heat of reaction can be appropriately removed. Conversely, by setting the space velocity to 15,000 $hr^{-1}$ or less, equipment such as a compressor can be of a practical size.

It is preferable to add 0.5 to 20 mol%, and more preferably 1 to 18 mol%, of water in the form of water vapor to the raw material gas. Though not to be bound by theory, it is believed that the presence of water in the reaction system suppresses the outflow of acetate from the catalyst. Since the addition of more than 20 mol% of water does not improve the effect described above and the hydrolysis of vinyl acetate may proceed, it is not preferable that a large amount of water be present in the raw material gas.

The material of the reactor is not particularly limited, and it is preferably a material having corrosion resistance.

The reaction temperature is preferably in the range of 100 to 300°C, and more preferably in the range of 120 to 250°C. When the reaction temperature is 100°C or higher, a suitable reaction rate can be maintained. When the reaction temperature is 300°C or lower, the heat of reaction can easily be removed.

The reaction pressure is preferably in the range of 0 to 3 MPaG (gauge pressure), and more preferably in the range of 0.1 to 1.5 MPaG. When the reaction pressure is 0 MPaG or higher, a suitable reaction rate can be maintained. When the reaction pressure is 3 MPaG or less, it is not necessary for equipment such as a reaction tube to have high-pressure resistance, whereby equipment costs can be suppressed.

Though it is preferable that high-purity ethylene be used as a reaction raw material, lower saturated hydrocarbons such as methane, ethane, and propane may be mixed therein.

The oxygen gas is not particularly limited. Gas diluted with an inert gas such as nitrogen gas or carbon dioxide gas can be supplied, for example, in the form of air. When a reaction gas is circulated, high concentration oxygen gas is generally used and oxygen gas having a purity of 99% or higher is advantageously used.

EXAMPLES

The present invention will be further described below by way of the Examples below, but is not limited to only these Examples.

Example 1 - Preparation of Catalyst A

A catalyst was prepared by the following procedure using a spherical silica carrier (sphere diameter: 5 mm, specific surface area: 155 $m^2$/g, water absorption: 0.85 g-water/g-carrier).

Step 1. 23.5 g of the carrier (water absorption amount: 20.0 g) was impregnated with an aqueous solution containing 2.3 g of $Na_2SiO_3 \cdot 9H_2O$ and having an amount corresponding to (0.95-fold the mass of) the water absorption amount of the carrier. The container containing the carrier and the aqueous solution was shaken to completely impregnate the solution and air-dried for 5 minutes. The water absorption amount was calculated from the carrier amount of 23.5 g and a water absorption of 0.85 g-water/g-carrier (the same applies to the following Examples and Comparative Examples).

Step 2. The carrier obtained in step 1 was immersed in an aqueous solution containing 0.92 g of $Na_2PdCl_4$, 0.28 g of $HAuCl_4$, and 0.04 g of copper chloride dihydrate and having twice the mass of the water absorption amount of the carrier, and allowed to stand at room temperature for 20 hours.

Step 3. 1.9 g of a 52% by mass hydrazine hydrate aqueous solution was added to the carrier dispersion obtained in step 2, gently mixed, and allowed to stand at room temperature for 4 hours. Next, the palladium/gold/copper/carrier composition obtained in the preceding step was washed with deionized water, and the washing was continued until the water after washing was free of chloride ions. The washed palladium/gold/copper/carrier composition was dried at 110°C for 4 hours.

Step 4. The palladium/gold/copper/carrier composition was impregnated with an aqueous solution containing 1.7 g of potassium acetate and having an amount equivalent to 0.9-fold the mass of the water absorption amount of the carrier, and dried at 110°C for 4 hours to obtain catalyst

A.

Example 2 - Preparation of Catalyst B

Catalyst B was obtained by repeating the operations of Example 1, except that in step 2 of Example 1, the amount of copper chloride dihydrate used was changed to 0.07 g.

Example 3 - Preparation of Catalyst C

Catalyst C was obtained by repeating the operations of Example 1, except that in step 2 of Example 1, the amount of copper chloride dihydrate used was changed to 0.09 g.

Example 4 - Preparation of Catalyst D

Catalyst D was obtained by repeating the operations of Example 1, except that in step 2 of Example 1, the amount of copper chloride dihydrate used was changed to 0.10 g.

Example 5 - Preparation of Catalyst E

Catalyst E was obtained by repeating the operations of Example 1, except that in step 2 of Example 1, the amount of copper chloride dihydrate used was changed to 0.11 g.

Example 6 - Preparation of Catalyst F

Catalyst F was obtained by repeating the operations of Example 1, except that in step 2 of Example 1, the amount of copper chloride dihydrate used was changed to 0.087 g and the amount of $HAuCl_4$ used was changed to 0.20 g.

Example 7 - Preparation of Catalyst G

Catalyst G was obtained by repeating the operations of Example 1, except that in step 2 of Example 1, the amount of copper chloride dihydrate used was changed to 0.087 g and the amount of $HAuCl_4$ used was changed to 0.27 g.

Comparative Example 1 - Preparation of Catalyst H

Catalyst H was obtained by repeating the operations of Example 1, except that in step 2 of Example 1, copper chloride dihydrate was not used.

Comparative Example 2 - Preparation of Catalyst I

Catalyst I was obtained by repeating the operations of Example 1, except that in step 2 of Example 1, $HAuCl_4$ was not used and the amount of copper chloride dihydrate used was changed to 0.14 g.

Comparative Example 3 - Preparation of Catalyst J

Using a spherical silica carrier (sphere diameter: 5 mm, specific surface area: 155 $m^2$/g, water absorption: 0.85 g-water/g-carrier), a catalyst was prepared by the following procedure in accordance with the descriptions in the Examples of Patent Literature 2 (JP 2002-516749 A).
Step i. 23.5 g of the carrier (water absorption amount: 20.0 g) was impregnated with an aqueous solution containing 0.32 g of $Na_2PdCl_4$ and 0.14 g of copper chloride dihydrate and having an amount corresponding to (0.95-fold the mass of) the water absorption amount of the carrier. The container containing the carrier and the aqueous solution was shaken to completely impregnate the solution.
Step ii. The carrier dispersion obtained in step i was brought into contact with a mixed solution of 0.7 g of a 50% by mass NaOH aqueous solution and 35.7 g of distilled water for 2.5 hours, and palladium and copper were immobilized on the carrier as palladium(II) and copper hydroxide.
Step iii. The palladium(II)/copper hydroxide/carrier composition obtained in step ii was washed with deionized water, and washing was continued until the water after washing was free of chloride ions. After washing with water, it was dried at a temperature of 150°C under nitrogen gas flow for 10 hours. Reduction treatment was then performed by contacting with ethylene (5 mol% in nitrogen gas) at 150°C for 5 hours in a gas phase. It was allowed to cool for 10 hours, washed with deionized water for 2 hours, and then dried in an oven at 150°C for 5 hours.
Step iv. 0.2 g of gold hydroxide was mixed with 0.10 g of potassium hydroxide in 17 mL of water, the resulting orange suspension was heated to 85°C, and all solids were dissolved for 5 hours to obtain a clear yellow solution of potassium aurate. The yellow solution was added to the palladium/copper/carrier composition obtained in step iii and allowed to impregnate the carrier therewith for 30 minutes. Thereafter, the composition was dried for 5 hours in an oven at 100°C under a nitrogen gas flow. It was then subjected to reduction treatment using ethylene (5 mol% in nitrogen gas) at a temperature of 120°C for 5 hours to obtain free metallic gold on the carrier.

Step v. The palladium/gold/copper/carrier composition obtained in step iv was impregnated with an aqueous solution containing 1.7 g of potassium acetate and having an amount equivalent to 0.9-fold the mass of the water absorption amount of the carrier and dried at 110°C for 4 hours to obtain catalyst J.

Comparative Example 4 - Preparation of Catalyst K

Catalyst K was obtained by repeating the operations of Examples 1 except that in step 2 of Example 1, copper chloride dihydrate was not used, and step 4 of Example 1 was changed so that 0.01 g of copper acetate monohydrate was dissolved together with 1.7 g of potassium acetate in water having an amount equivalent to 0.9-fold the mass of the water absorption amount of the carrier.

[Catalyst Evaluation]

<Measurement of Metal (Copper, Palladium, and Gold) and Potassium Acetate Support Amounts>

3 g of the support catalyst sample was pulverized and pressed into a disc having an inner diameter of 3 cm. The amounts of metals in this disc were measured with a fluorescent X-ray spectrometer PW2404 manufactured by Philips. For the potassium acetate, the amount of potassium atoms was quantified and converted into a potassium acetate amount.

<Catalyst Activity Evaluation Test>

6.7 mL of a catalyst was diluted with 75 mL of glass beads and filled in a reaction tube (composed of SUS316L, inner diameter: 22 mm, length: 480 mm). Under the conditions of a reaction temperature of 150°C and a reaction pressure of 0.6 MPaG, a gas having a gas composition of $C_2H_4/O_2/H_2O/HOAc/N_2$ = 45/6/5/23/21 (mol%) was flowed at a flow rate of 66.7 NL/h for 240 hours to stabilize the catalyst state. Thereafter, the gas flow rate was changed to 20.0 NL/h, and the reaction temperature was further changed to 145°C, 150°C, 155°C or 160°C to evaluate catalyst activity and selectivity. When the gas flow rate and reaction temperature were changed, catalyst activity and selectivity were evaluated after stabilizing the conditions of the apparatus and catalyst for at least 4 hours. In general, the higher the vinyl acetate activity (STY) (g/L-cat·h), the lower the vinyl acetate selectivity. Thus, the vinyl acetate selectivity was compared at the same vinyl acetate activity for each catalyst. For example, the vinyl acetate selectivity at a vinyl acetate activity of 550 (g/L-cat·h) was calculated by interpolating from a polynomial fitted curve consisting of plots of vinyl acetate activity and vinyl acetate selectivity at each reaction temperature.
The reactor outlet gas was analyzed using the following method.

1. Oxygen

Using the absolute calibration curve method, 50 mL of the effluent gas was sampled, and the entire amount was flowed into a 1 mL gas sampler attached to the gas chromatography and analyzed under the following conditions.

Gas chromatograph: Gas Chromatograph (GC-14B manufactured by Shimadzu Corporation) having a Shimadzu Gas Chromatograph Gas Sampler (MGS-4: measuring tube: 1 mL)
Column: MS-5A IS 60/80 mesh (3 mmΦ × 3 m)
Carrier gas: Helium (flow rate: 20 mL/min)
Temperature conditions: Detector temperature, vaporization chamber temperature: 110°C, column temperature: constant 70°C
Detector: TCD (He pressure: 70 kPaG, current: 100 mA)

2. Acetic Acid

Using the internal standard method, 1 mL of 1,4-dioxane was added as an internal standard to 10 mL of the reaction liquid, and 0.2 μL of this was injected as an analysis liquid and analyzed under the following conditions.

Gas chromatograph: GC-14B manufactured by Shimadzu Corporation
Column: Packed column Thermon-3000 (length: 3 m, inner diameter: 0.3 mm)
Carrier gas: Nitrogen (flow rate: 20 mL/min)
Temperature conditions: Detector temperature, vaporization chamber temperature: 180°C, column temperature: held at 50°C for 6 minutes from the start of analysis, then raised to 150°C at a rate of 10°C/min and held at 150°C for 10 minutes
Detector: FID (H$_2$ pressure: 40 kPaG, air pressure: 100 kPaG)

3. Vinyl Acetate

Using the internal standard method, 1 g of 1,4-dioxane was added as an internal standard to 6 g of the reaction solution, and 0.3 μL of this was injected as an analysis liquid and analyzed under the following conditions.

Gas chromatograph: GC-9A manufactured by Shimadzu Corporation
Column: Capillary column TC-WAX (length: 30 m, inner diameter: 0.25 mm, membrane thickness: 0.5 μm)
Carrier gas: Nitrogen (flow rate: 30 mL/min)
Temperature conditions: Detector temperature, vaporization chamber temperature: 200°C, column temperature: held at 45°C for 2 minutes from the start of analysis, then raised to 130°C at a rate of 4°C/min and held at 130°C for 15 minutes. Thereafter, the temperature was raised to 200°C at a rate of 25°C/min and held at 200°C for 10 minutes
Detector: FID (H$_2$ pressure: 60 kPaG, air pressure: 100 kPaG)

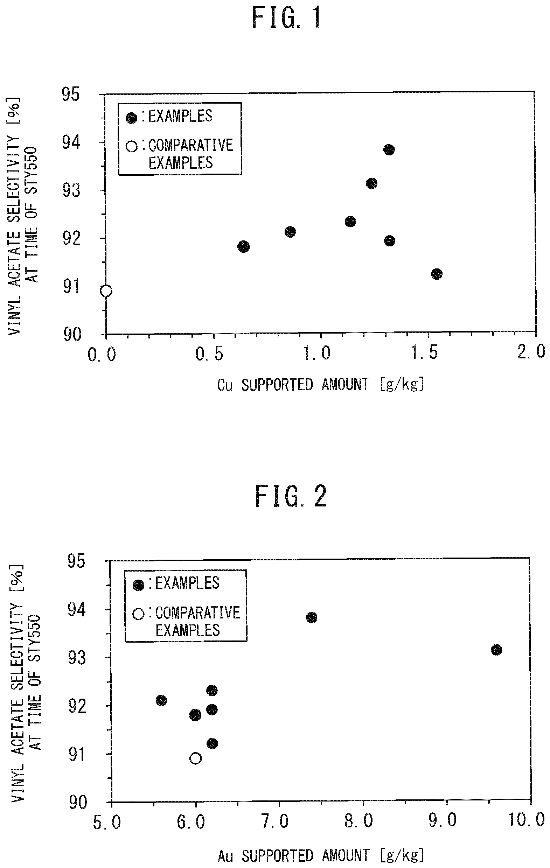

The catalyst evaluation results are shown in Table 1 and FIGS. 1 and 2. Note that in FIGS. 1 and 2, Comparative Examples 2, 3, and 4 are not plotted because their STYs were significantly low. Vinyl acetate selectivity is based on ethylene.

[Table 1]

| Catalyst | Example/ Comp Ex | Metal component in catalyst [g/kg] | | | Vinyl acetate selectivity [%][1] | Vinyl acetate activity [g/L-cat·h][2] |
|---|---|---|---|---|---|---|
| | | Cu | Pd | Au | | |
| Catalyst A | Ex 1 | 0.64 | 12.0 | 6.0 | 91.8 | 539 |
| Catalyst B | Ex 2 | 0.86 | 11.6 | 5.6 | 92.1 | 525 |
| Catalyst C | Ex 3 | 1.14 | 12.2 | 6.2 | 92.3 | 529 |
| Catalyst D | Ex 4 | 1.32 | 11.8 | 6.2 | 91.9 | 492 |
| Catalyst E | Ex 5 | 1.54 | 11.8 | 6.2 | 91.2 | 502 |
| Catalyst F | Ex 6 | 1.32 | 12.2 | 7.4 | 93.8 | 534 |
| Catalyst G | Ex 7 | 1.24 | 11.6 | 9.6 | 93.1 | 466 |
| Catalyst H | Comp Ex 1 | 0.00 | 12.2 | 6.0 | 90.9 | 546 |
| Catalyst I | Comp Ex 2 | 1.98 | 12.4 | 0.0 | 91.0 | 58 |
| Catalyst J | Comp Ex 3 | 1.12 | 12.0 | 6.2 | 90.5 | 358 |
| Catalyst K | Comp Ex 4 | 1.16 | 12.2 | 6.2 | 91.3 | 410 |
| 1) values at a vinyl acetate activity (STY) [g/L-cat·h] of 550 for catalysts A to H and due to remarkably low vinyl acetate activity, values at a reaction temperature of 150°C for catalysts I to K  2) values at a reaction temperature of 150°C | | | | | | |

Comparing Examples 1 to 7 with Comparative Example 1, by supporting copper, the vinyl acetate selectivity is improved by 0.3 to 2.9 points, which is a remarkable effect. Focusing on Examples 4 and 6, it can be understood that the vinyl acetate selectivity is further improved by increasing the amount of supported gold even with the same amount of supported copper. In Comparative Example 2, in which gold was not supported and a combination of palladium and copper was supported, the vinyl acetate activity (g/L-cat·h) did not reach 550 even at a reaction temperature of 160°C, and the activity was extremely low. In Comparative Examples 3 and 4, catalysts to which the copper-containing compound was added in a step different from the palladium-containing compound and the gold-containing compound were evaluated, and the vinyl acetate activity was clearly lower than that of Examples 1 to 7, and the vinyl acetate selectivity was also lower than that of all the Examples except Example 5. From the foregoing, it can be understood that by using the preparation method of Examples 1 to 7, in which not only palladium, gold, and copper are combined, but also the palladium-containing compound, the gold-containing compound, and the copper-containing compound are supported while reacting with an alkali in the same step, exceptionally high vinyl acetate selectivity could be obtained while ensuring high vinyl acetate activity. From Examples 1 to 7, it can be demonstrated that the amount of supported Cu is preferably 0.1 to 1.6 g/kg, and the amount of supported Au is preferably 4.0 to 12.0 g/kg.

INDUSTRIAL APPLICABILITY

The present invention can provide a vinyl acetate production catalyst having excellent selectivity while ensuring high catalytic activity, and is industrially useful.

**Claims**

1. A method for producing a vinyl acetate production catalyst comprising a carrier, copper, palladium, gold, and an acetate, the method comprising the following steps in this order:

   step 1: impregnating a carrier with an alkaline solution,
   step 2: contacting and impregnating the carrier with a solution containing a copper-containing compound, a palladium-containing compound, and a gold-containing compound,
   step 3: performing a reduction treatment, and
   step 4: supporting the acetate on the carrier.

2. The method according to claim 1, wherein a mass of supported metallic copper per kg of catalyst is 0.1 g or more and 1.6 g or less.

3. The method according to claim 1 or 2, wherein a mass of supported metallic palladium per kg of catalyst is 8.0 g or more and 16.0 g or less.

4. The method according to any one of claims 1 to 3, wherein a mass of supported metallic gold per kg of catalyst is 4.0 g or more and 12.0 g or less.

5. The method according to any one of claims 1 to 4, wherein a mass of supported acetate per kg of catalyst is 40 g or more and 100 g or less.

6. A method for producing vinyl acetate using ethylene, oxygen, and acetic acid as raw materials, wherein a vinyl acetate production catalyst obtained by the method according to any one of claims 1 to 5 is used.

# FIG. 1

# FIG. 2

## INTERNATIONAL SEARCH REPORT

| International application No. |
|---|
| **PCT/JP2021/028834** |

**A.    CLASSIFICATION OF SUBJECT MATTER**

***B01J 23/89***(2006.01)i; ***B01J 37/02***(2006.01)i; ***B01J 37/16***(2006.01)i; ***C07C 67/055***(2006.01)i; ***C07C 69/15***(2006.01)i; *C07B 61/00*(2006.01)n
FI:    B01J23/89 Z; B01J37/02 101D; B01J37/16; C07C67/055; C07C69/15; C07B61/00 300

According to International Patent Classification (IPC) or to both national classification and IPC

**B.    FIELDS SEARCHED**

Minimum documentation searched (classification system followed by classification symbols)

B01J21/00-38/74; C07C67/055; C07C69/15; C07B61/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C.    DOCUMENTS CONSIDERED TO BE RELEVANT**

| Category* | Citation of document, with indication, where appropriate, of the relevant passages | Relevant to claim No. |
|---|---|---|
| X | JP 08-510685 A (ENGELHARD CORP.) 12 November 1996 (1996-11-12)<br>    examples 1-3, 8, 9, 12-23, 25-27, claims, p. 10, line 14 to p. 17, line 10, p. 30, line 17 to p. 33, line 17 | 1-6 |
| A | JP 2008-080326 A (SHOWA DENKO K.K.) 10 April 2008 (2008-04-10)<br>    entire text | 1-6 |
| A | JP 2003-513788 A (CELANESE INTERNATIONAL CORP.) 15 April 2003 (2003-04-15)<br>    entire text | 1-6 |
| A | JP 2014-061516 A (CLARIANT INTERNATIONAL LTD.) 10 April 2014 (2014-04-10)<br>    entire text, all drawings | 1-6 |

☐ Further documents are listed in the continuation of Box C.          ☑ See patent family annex.

| *   Special categories of cited documents: | "T"   later document published after the international filing date or priority date and not in conflict with the application but cited to understand the principle or theory underlying the invention |
|---|---|
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **15 September 2021** | **28 September 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/028834**

| Patent document cited in search report | | | Publication date (day/month/year) | Patent family member(s) | | | Publication date (day/month/year) |
|---|---|---|---|---|---|---|---|
| JP | 08-510685 | A | 12 November 1996 | US | 5347046 | A | |
| | | | | examples 1-3, 8, 9, 12-23, 25-27, claims, column 3, line 15 to p. 7, line 27, column 15, line 29 to column 16, line 42 | | | |
| | | | | WO | 1994/027720 | A1 | |
| | | | | EP | 708684 | A1 | |
| | | | | AU | 6912994 | A | |
| | | | | BR | 9406696 | A | |
| | | | | CA | 2161952 | A | |
| | | | | KR | 10-1996-0702348 | A | |
| | | | | CN | 1123527 | A | |
| | | | | TW | 254934 | B | |
| JP | 2008-080326 | A | 10 April 2008 | US | 2010/0168465 | A1 | |
| | | | | entire text | | | |
| | | | | WO | 2008/029597 | A1 | |
| | | | | EP | 2059340 | A1 | |
| | | | | KR | 10-2009-0025352 | A | |
| | | | | CN | 101511473 | A | |
| | | | | TW | 200831185 | A | |
| JP | 2003-513788 | A | 15 April 2003 | WO | 2001/036091 | A1 | |
| | | | | entire text | | | |
| | | | | EP | 1237650 | A1 | |
| | | | | BR | 15179 | A | |
| | | | | CA | 2389448 | A | |
| | | | | CN | 1391496 | A | |
| | | | | KR | 10-2002-0048435 | A | |
| JP | 2014-061516 | A | 10 April 2014 | US | 2014/0081041 | A1 | |
| | | | | entire text, all drawings | | | |
| | | | | DE | 102013015436 | A | |
| | | | | TW | 201424841 | A | |

Form PCT/ISA/210 (patent family annex) (January 2015)

**REFERENCES CITED IN THE DESCRIPTION**

**Patent documents cited in the description**

- JP 2008080326 A

- JP 2002516749 A